# EUROPEAN PATENT SPECIFICATION

(11) **EP 1 992 353 B1**
(45) Date of publication and mention of the grant of the patent: **07.05.2014**
(21) Application number: 07714138.0
(22) Date of filing: 14.02.2007
(51) Int. Cl.: C07K 5/083, A23L 1/305, A61K 38/06

(54) **VASODILATOR**
VASODILATATOR
VASODILATATEUR

(30) Priority: 14.02.2006 JP 2006035944
(43) Date of publication of application: 19.11.2008
(73) Proprietor: Calpis Co., Ltd., Shibuya-ku, Tokyo 150-0022 (JP)
(72) Inventor: HIROTA, Tatsuhiko, Sagamihara, Kanagawa 229-0006 (JP); OHKI, Kohji, Sagamihara-shi, Kanagawa 229-0006 (JP)
(74) Representative: Hallybone, Huw George
(86) International application number: PCT/JP2007/052569
(87) International publication number: WO 2007/094340

(56) References cited:
- EP-A- 0 162 032
- EP-A- 1 016 709
- WO-A1-00/41572
- WO-A1-99/16862
- JP-A- 06 040 944
- JP-A- 2003 513 636
- PRASAD A. ET AL.: 'Abnormal flow-mediated epicardial vasomotion in human coronary arteries is improved by angiotensin-converting enzyme inhibition' JOURNAL OF THE AMERICAN COLLEGE OF CARDIOLOGY vol. 33, no. 3, 1999, pages 796 - 804, XP003016680
- AI SAIGA ET AL: "Action Mechanism of an Angiotensin I-Converting Enzyme Inhibitory Peptide Derived from Chicken Breast Muscle", JOURNAL OF AGRICULTURAL AND FOOD CHEMISTRY (AMERICAN CHEMICAL SOCIETY), vol. 54, no. 3, 1 February 2006 (2006-02-01), pages 942-945, XP055056812, St Louis Mi USA ISSN: 0021-8561, DOI: 10.1021/jf0508201
- B. PITT: "Effect of ACE inhibitors on endothelial dysfunction: Unanswered questions and implications for further investigation and therapy", CARDIOVASCULAR DRUGS AND THERAPY, vol. 10, no. 4, 1996, pages 469-473, Boston MA USA
- W. KIOWSKI ET AL: "Effects of Cilazapril on Vascular Structure and Function in Essential Hypertension", HYPERTENSION, vol. 27, no. 3, 1996, pages 371-376, Dallas TX USA
- B. ERZEN ET AL.: "Treatment of Essential Arterial Hypertension with Enalapril Does Not Result in Normalization of Endothelial Dysfunction of the Conduit Arteries", ANGIOLOGY, vol. 57, no. 2, 1 March 2006 (2006-03-01), pages 187-192, Thousand Oaks, CA USA
- M. Sipola: "EFFECTS OF MILK PRODUCTS AND MILK PROTEIN-DERIVED PEPTIDES ONBLOOD PRESSURE AND ARTERIAL FUNCTIONIN RATS", 15 March 2002 (2002-03-15), University of Helsinki, Helsinki, XP055033903, pages 1-85,
- YI-XIN WANG ET AL: "Increased aortic stiffness assessed by pulse wave velocity in apolipoprotein E-deficient mice", AMERICAN JOURNAL OF PHYSIOLOGY: HEART AND CIRCULATORY PHYSIOLOGY, AMERICAN PHYSIOLOGICAL SOCIETY, US, vol. 278, 1 February 2000 (2000-02-01), pages H428-H434, XP002489146, ISSN: 0363-6135
- VAN POPELE N M ET AL: "Association between arterial stiffness and atherosclerosis - The Rotterdam Study", STROKE, LIPPINCOTT WILLIAMS & WILKINS, US, no. 32, 4 October 2000 (2000-10-04), pages 454-460, XP003028360, ISSN: 0039-2499
- M.A. CREAGER & M.A. RODDY: "Effect of captopril and enalapril on endothelial function in hypertensive patients", HYPERTENTION (AMERICAN HEART ASSOCIATION), vol. 24, 1994, pages 499-505, Dallas TX USA

## Description

The present invention relates to a vasodilator having an endothelium-dependent vasodilator effect, and functional food having such an effect.

Ischemic diseases, such as myocardial or cerebral infarction, which are the ultimate development of arteriosclerotic diseases, account for major part of the cause of death in Japan, along with cancers. Risk factors for arteriosclerosis include hyperlipemia, hyperlipidemia, hypertension, diabetes, smoking, obesity, hyperuricemia, aging, stress, and the like, which are interrelated to cause angiopathy. Thus even if each risk factor is low, cumulation of the factors additively and synergistically increases the risk, which also increases the risk of ischemic diseases.

On the other hand, it is envisaged that mere mitigation of one of the above risk factors will not present onset of arteriosclerosis. For example, Non-patent Publications 1 and 2 report the absence of interrelationship between the blood cholesterol level and onset of arteriosclerosis, Non-patent Publication 3 teaches that suppression of hypertension does not change the degree of arteriosclerosis, and Non-patent Publication 4 describes that administration of an angiotensin converting enzyme inhibitor containing enalapril as an active component does not result in an arteriosclerosis inhibitory effect. Further, when infarcted, ischemic diseases, for example, acute heart failure is developed, according to Non-patent Publication 5, medicament, such as a vasodilator including nitroprusside or nitroglycerine, a diuretic, or a cardiotonic, is needed to stabilize the hemodynamics.

Thus, though Patent Publications 1 and 2, for example, disclose that tripeptides Val-Pro-Pro and Ile-Pro-Pro have an angiotensin I converting enzyme inhibitory activity, which leads to a hypotensive effect, and also an anti-stress effect, this does not mean that these tripeptides have an anti-arteriosclerotic effect or a vasodilator effect.

Arteriosclerosis is a pathology wherein the arterial wall is thickened to loose its elasticity. One of the factors for such symptom is recently considered to be injury or decreased functions of vascular endothelial cells.

Thus an endothelium-dependent vasodilator is expected to have an inhibitory effect on arteriosclerosis.
Patent Publication 1: JP-6-197786-A
Patent Publication 2: JP-11-100328-A
Non-patent Publication 1: Shoku no Kagaku 257 (1999), p20-25
Non-patent Publication 2: Atherosclerosis 151 (2000), p501-508
Non-patent Publication 3: Circulation 104 (2001), p2391-2394
Non-patent Publication 4: International Journal of Cardiology 81 (2001), p107-115
Non-patent Publication 5: Bessatsu Igaku no Ayumi, Junkanki Shikkann, state of arts ver. 2 (2001), p332-334

JP 06 040 944 A teaches how to obtain an industrially effective angiotensin converting enzyme inhibitor capable of exhibiting excellent activity in inhibiting the angiotensin converting enzyme by a very small amount of oral administration, inexpensively and readily producable. The angiotensin converting enzyme inhibitor contains a peptide containing Val-Pro-Pro and having 3-10 amino acid residues as an active ingredient. This inhibitor is produced by subjecting a food raw material containing a peptide composed of Val-Pro-Pro to fermentation treatment with lactic acid bacteria.

WO 99 16862 A discloses a strain of a lactic bacterium capable of producing a large amount of lactotripeptide and a fermented milk product which contains a number of components having hypotensive and stress relaxation activities and can be easily eaten and swallowed. More specifically a lactic bacterium belonging to the genus Lactobacillus helveticus, i.e. Lactobacillus helveticus strain CM4 (deposited under the accession number of PERM PB-6060 with National Institute of Bioscience and Human-Technology), which is characterized by having particular mycological properties, producing tripeptides Val-Pro-Pro and/or Ile-Pro-Proin an amount of not less than 60 µg/ml in terms of Val-Pro-Prowhen cultured in animal milk as the medium, containing not less than 9 % by weight (in terms of solid content) of non-fat milk, and exhibiting an extracellular protease activity of not less than 400 U/OD590; and a fermented milk product prepared by fermenting animal milk with the above lactic bacterium.

WO 00 41572 A1 discloses processes for producing fermented milk and milk serum whereby fermented milk and milk serum containing an ACEI peptide at a high content, which have high safety and are usable as drugs, functional foods, health foods, etc., can be efficiently obtained at a high yield. A process for preparing fermented milk containing curd pieces and milk serum containing an angiotensin converting enzyme inhibitory peptide which involves the step of mixing and stirring a milk-containing material with lactic acid bacteria to give a mixed material, and the step of fermenting the mixed material under stirring so as to form the milk serum containing the angiotensin converting enzyme inhibitory peptide; and a process for producing milk serum further involving the step of centrifuging and/or compression-filtering the fermented milk obtained above to thereby separate and collect the milk serum.

JP 2003 513636 A discloses a process for preparing a product containing antihypertensive peptides by fermenting a casein-containing starting material with lactic acid bacteria. The invention also relates to the obtained product and its use as a functional product as such or as an ingredient or additive of edible substances.

Prasad, A., et al., Journal of the American College of Cardiology, 1999, 33(3), 796-804 discloses a study performed to determine whether angiotensin converting enzyme (ACE) inhibition improved endothelium-dependent flow-mediated vasodilation in patients with atherosclerosis or its risk factors and whether this is mediated by anhanced bradykinin activity.

EP-A-1 016 709 teaches a fermented milk product that contains lactic acid bacteria capable of producing a large amount of lactotripeptide and a large amount of active ingredient having hypotensive activity and anti-stress effect, and that can be taken pleasantly as foods or beverages. Lactic acid bacteria of Lactobacillus helveticus having specific bacteriological properties, the bacteria, when cultured in a medium of animal milk containing 9wt% solid of non-fat milk, producing tripeptides Val-Pro-Pro and Ile-Pro-Proin an amount of 60 mu g in terms of Val-Pro-Pro per ml medium, and the bacteria exhibiting extracellular proteinase activity of not lower than 400U/0D590. Lactobacillus helveticus CM4 strain (deposited at National Institute of Bioscience and Human-Technology Agency of Industrial Science and Technology, deposition number FERM BP-6060).; A fermented milk product obtained by fermenting an animal milk with these lactic acid bacteria.

EP-A-0 162 032 discloses a novel high molecular weight atrial peptide having useful natriuredc, diuretic and vasodilating activity.

It is an object of the present invention to provide a vasodilator having an endothelium-dependent vasodilator effect as defined in claim 1.

According to the present invention, there is provided a vasodilator comprising at least one of peptides Val-Pro-Pro and Ile-Pro-Pro as an active component as defined in claim 1.

Containing, as an active component, Val-Pro-Pro and/or Ile-Pro-Pro derived from animal milk casein or the like, or a proteolytic product containing at least one of these peptides, the vasodilator according to the present invention are excellent in safety, and have an endothelium-dependent vasodilator effect.

The vasodilator according to the present invention is useful for ensuring blood circulation when a patient is suffered from an ischemic disease, such as cardiac or cerebral infarction, relaxes blood vessels which are more prone to constriction due to aging, lifestyle-related diseases or the like, and may be expected to prevent arteriosclerosis, or neck stiffness, cold constitution, thrombosis, or the like symptoms associated with blood flow dysfunction_{.}
Fig. 1 is a graph showing the results of vasodilating tests conducted in Example 1 and Comparative Example 1.
Fig. 2 is a graph showing the results of a confirmatory test for the endothelium dependency of the vasodilator response conducted in Example 1.

The present invention will now be explained in detail.

The vasodilator according to the present invention contains, as an active component, val-Pro-Pro and/or Ile-Pro-Pro (these tripeptides are abbreviated as VPP and IPP, respectively, hereinbelow) or a proteolytic product containing at least one of these tripeptides.

The tripeptides may be those to which a pharmacologically acceptable salt has been added, including salts of inorganic acids, such as hydrochlorides, sodium salts, and phosphates, or salts of organic acids, such as citrates, maleates, fumarates, tartrates, and lactates.

The tripeptides may be prepared by digesting and purifying peptides or proteins containing the amino acid sequence VPP and/or IPP through fermentation with microorganisms, by enzymatic hydrolysis of such peptides or proteins, or by synthesis. For detail, see Patent Publications 1 and 2 mentioned above. Thus, the active component of the present invention may be a fermentation product containing at least one of the peptides VPP and IPP obtained by fermentation of peptides or proteins containing the amino acid sequence VPP and/or IPP with microorganisms, or a purified product thereof, or a hydrolysate containing at least one of the peptides VPP and IPP obtained by digesting peptides or proteins containing the amino acid sequence VPP and/or IPP with enzymes, or a purified product thereof.

The effective dose of the vasodilator of the present invention is usually 10 µg to 10 g, preferably about 1 mg to 1 g per day for human in terms of the tripeptides, for achieving the effect in a single dose.

The administration schedule of the vasodilator may be adjusted to the symptoms of a disease. For acute symptoms, single or continuous parenteral administration is suitable. For chronic symptoms or for prophylactic use, regular oral administration for 30 days or longer is preferred.

The administration route of the vasodilator according to the present invention may either be oral or parenteral. The parenteral administration may be topical, transdermal, intravenous, intramuscular, subcutaneous, intradermal, intraperitoneal, intrathoracic, or intraspinal administration. Direct administration to the diseased area is also possible.

The form of the vasodilator according to the present invention may be decided depending on the administration route, and may be in the form of a formulation, such as tablets, pills, hard capsules, soft capsules, microcapsules, powders, granules, liquids, suspensions, or emulsions.

The formulationmaybemadewith, for example, acarrier, adjuvant, excipient, auxiliary excipient, antiseptic, stabilizer, binder, pH regulator, buffer, thickener, gelatinizer, preservative, anti-oxidant, or the like which are acceptable for pharmaceutical use, as desired, in a unit dose form that is required in generally approved formulation.

The present invention will now be explained in more detail with reference to Examples, which are illustrative only and do not intend to limit the present invention.

### Synthesis Example

IPP and VPP were synthesized through the following organic chemical synthesis by the solid phase method in an automated peptide synthesizer (PSSM-8) manufactured by SHIMADZU CORPORATION.

50 mg of 2-chlorotrityl polystyrene resin to which proline having its amino group protected with a fluorenylmethyloxycarbonyl group (abbreviated as Fmoc hereinbelow) was bound (registered trademark SynProPep Resin, manufactured by SHIMADZU CORPORATION), was used as a solid support. 100 µmol each of Fmoc-Ile, Fmoc-Pro, and Fmoc-Val, wherein the amino groups were protected with the Fmoc group, were sequentially reacted by a routine method according to the amino acid sequence mentioned above to obtain a peptide-bound resin.

Thepeptide-boundresinwas suspended in 1 ml of reaction liquid A (10 vol% acetic acid, 10 vol% trifluoroethanol, and 80 vol% dichloromethane), reacted at room temperature for 30 to 60 minutes to cleave the peptides from the resin, and filtered through a glass filter. The solvent in the resulting filtrate was removed under reduced pressure, and immediately 1 ml of reaction liquid B (82.5 vol% trifluoroacetic acid, 3 vol% ethyl methyl sulfide, 5 vol% purified water, 5 vol%thioanisol, 2.5 vol% ethanedithiol, and 2 vol% thiophenol) was added. The resulting mixture was reacted at room temperature for 6 hours to remove the side chain protective groups, to which 10 ml of anhydrous ether was added to precipitate the peptides. The precipitate was separated by centrifugation at 3000 rpm for 5 minutes, washed several times with anhydrous ether, and dried under nitrogen gas. All of the crude synthesized peptides thus obtained was dissolved in 2 ml of a 0.1 N aqueous solution of hydrochloric acid, and purified by C18 reverse phase HPLC under the following conditions. Pump: model L6200 intelligent pump (manufactured by HITACHI, LTD.); Detector: ultraviolet absorption at 215 nm was detected with model L4000 UV detector (manufactured by HITACHI, LTD.); Column: µBondasphere 5µ C18 (manufactured by Nihon Waters K.K.); Eluate: Liquid A of a 0.1 wt% TFA aqueous solution and Liquid B of 0.1 wt% TFA-containing acetonitrile, (B/A+B)x100 (%): 0 to 40 % (over 60 min); Flow rate: 1 ml/min.

The eluted fraction having the maximum absorption was taken out and lyophilized to obtain the objective synthesized peptides Ile-Pro-Pro and Val-Pro-Pro at the yields of 5.7 mg and 6.5 mg, respectively. The purified peptides were analyzed from the N-terminal in an automated protein sequencer (model PPSQ-10, manufactured by SHIMADZU CORPORATION), and further analyzed in an amino acid analyzer (model 800 series, manufactured by JASCO CORPORATION). It was confirmed that the peptides were prepared as designed.

### Example 1

### <Vasodilating Test>

The thoracic aorta of a Wistar rat was taken out, cut into 2 mm long, and made into an aorta ring. The ring was set in a Magnus apparatus (product name "micro tissue organ bath MTB-1Z", manufactured by LABO SUPPORT CO., LTD.), and allowed to equilibrate with a constant tension. The constriction response of the aorta ring was confirmed with 50 mM KCl. The aorta ring was then allowed to constrict with 1 µM of phenylephrine, and the stably constricted samples were observed for endothelium-dependent vasodilator response using 1 µM of acetylcholine to confirm that the endothelial functions were maintained.

Next, the aorta ring was preliminarily constricted with 1 µM phenylephrine, and VPP and IPP prepared in Synthesis Example were added at ten-fold increasing concentrations from 10⁻⁹ M to observe the vasodilator response through the change in tension of the aorta ring. The dose dependency of the vasodilator response was also studied.

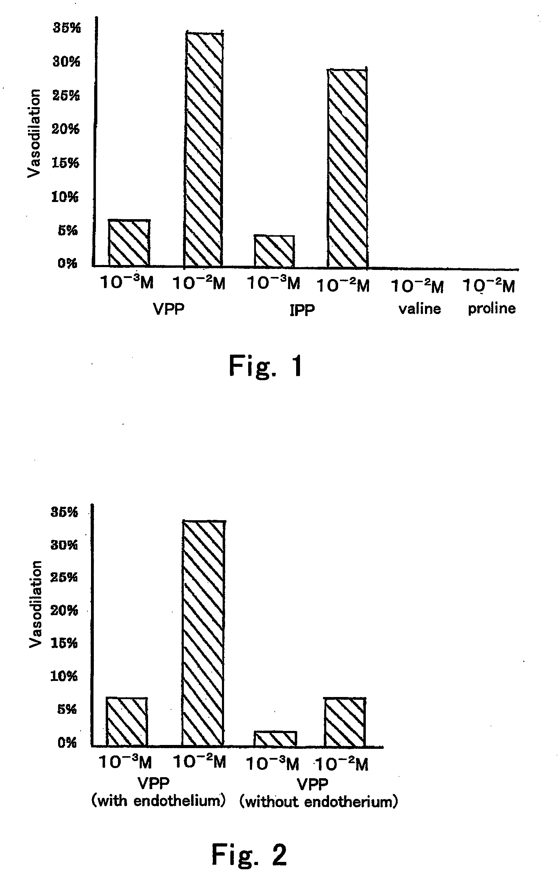

The result was that the vasodilator response to VPP and IPP was observed from the concentration of 1 mM, and a clear, transient vasodilator response was confirmed at 10 mM. The results are shown in Fig. 1.

### <Confirmatory Test for Endothelium Dependency of Vasodilator Response to VPP and IPP>

In order to confirm that the vasodilator effect of VPP and IPP was associated with the vascular endothelium, a vasodilating test similar to the above was conducted with VPP, using as a control a blood vessel from which the vascular endothelium was physically removed by a routine method. The result was that the vasodilator response was weakened due to the removal of the endothelium, indicating that the vasodilating effect of VPP and IPP was highly endothelium dependent. The results are shown in Fig. 2.

### Comparative Example 1

The vasodilating test was conducted in the same way as in Example 1, except that the tripeptides VPP and IPP were replaced with amino acids, valine (Val) and proline (Pro), at the same concentration.

The results were that no vasodilator response was observed at 10 mM with either Val or Pro. The results are shown in Fig. 1.

From the results discussed above, it is understood that VPP and IPP are active as vasodilators in the peptide forms.

### Referential Example

JP-2004-244359-A reports a vasodilating medical composition and a vasodilating health foods composition containing, as an active component, peptides obtained by hydrolyzing proteins derived from various milk proteins. Based on the conventional knowledge that β-casein and κ-casein have the amino acid sequences including VPP and IPP, the peptides obtained in the Production Examples disclosed in this publication were measured for VPP and IPP.

Commercially available skim milk, which is a protein material containing β-casein and κ-casein was tested among the material used in the Production Examples. 1 kg of the skim milk was suspended in 2 L of warm water, adjusted to pH 7.5, mixed with 40 g of thermoase (manufactured by DAIWA FINE CHEMICALS CO., LTD.), and reacted at 50 °C for 16 hours. After the reaction, the reaction liquid was heated at 100 °C for 10 minutes for inactivating the enzyme, to obtain hydrolyzed peptides. Through the analysis with a high performance liquid chromatograph-mass spectrometer, it was confirmed that the obtained hydrolyzed peptides did not include VPP or IPP.

From the results discussed above, it was demonstrated that the vasodilation reported in JP-2004-244359-A was caused by components other than VPP and IPP.

## Claims

1. Val-Pro-Pro and/or Ile-Pro-Pro for use to achieve endothelium-dependent vasodilation in the treatment or prophylaxis of cardiac infarction, cerebral infarction, arteriosclerosis, neck stiffness associated with blood flow dysfunction, or cold constitution associated with blood flow dysfunction.

2. Val-Pro-Pro and/or Ile-Pro-Pro for use as claimed in claim 1, in the treatment of cardiac infarction or cerebral infarction.

3. Val-Pro-Pro and/or Ile-Pro-Pro for use as claimed in claim 1, in the prophylaxis of arteriosclerosis, neck stiffness associated with blood flow dysfunction, cold constitution associated with blood flow dysfunction, or thrombosis associated with blood flow dysfunction.

## Patentansprüche

1. Val-Pro-Pro und/oder Ile-Pro-Pro zur Verwendung zur Erzielung einer endothelabhängigen Gefäßerweiterung bei der Behandlung oder Prophylaxe von Herzinfarkt, Hirninfarkt, Arteriosklerose, Nackensteifheit, assoziiert mit einer Störung des Blutflusses oder kalter Konstitution, assoziiert mit einer Störung des Blutflusses.

2. Val-Pro-Pro und/oder Ile-Pro-Pro zur Verwendung, wie in Anspruch 1 beansprucht, zur Behandlung von Herzinfarkt oder Hirninfarkt.

3. Val-Pro-Pro und/oder Ile-Pro-Pro zur Verwendung, wie in Anspruch 1 beansprucht, zur Prophylaxe von Arteriosklerose, Nackensteifheit, assoziiert mit einer Störung des Blutflusses, kalter Konstitution, assoziiert mit einer Störung des Blutflusses oder Thrombose, assoziiert mit einer Störung des Blutflusses.

## Revendications

1. Val-Pro-Pro et/ou Ile-Pro-Pro destiné à être utilisé pour obtenir une vasodilatation dépendante de l'endothélium dans le traitement ou la prophylaxie d'un infarctus cardiaque, d'un infarctus cérébral, de l'artériosclérose, d'une raideur du cou associée à un dysfonctionnement de la circulation sanguine, ou d'une constitution froide associée à un dysfonctionnement de la circulation sanguine.

2. Val-Pro-Pro et/ou Ile-Pro-Pro destiné à être utilisé selon la revendication 1, dans le traitement d'un infarctus cardiaque ou d'un infarctus cérébral.

3. Val-Pro-Pro et/ou Ile-Pro-Pro destiné à être utilisé selon la revendication 1, dans la prophylaxie de l'artériosclérose, d'une raideur du cou associée à un dysfonctionnement de la circulation sanguine, d'une constitution froide associée à un dysfonctionnement de la circulation sanguine, ou d'une thrombose associée à un dysfonctionnement de la circulation sanguine.
